# EUROPEAN PATENT APPLICATION

(11) **EP 3 078 958 A2**
(43) Date of publication of application: **12.10.2016**
(21) Application number: 16160585.2
(22) Date of filing: 16.03.2016
(51) Int. Cl.: G01N 21/03, G01N 21/31, B01L 3/00

(54) **ARRANGEMENT AND METHOD FOR STUDYING SAMPLES AND CONTAINER FOR SAMPLES**

(30) Priority: 16.03.2015 FI 20155176
(71) Applicant: MicroAtmos Oy, 24100 Salo (FI)
(72) Inventor: Salminen, Eeva, 24100 Salo (FI)
(74) Representative: Kolster Oy Ab

(57) **Abstract**

The invention relates to an arrangement for studying samples and a substrate for samples. The arrangement comprises a frame (1) providing a basis for at least two samples. The frame (1) may be provided with at least two light sources (4a - 4i) for emitting light. The arrangement further comprises at least one substrate (7) for samples and at least two sensors (8) for detecting how much the samples emit or absorb light. The substrate (7) may comprise a bottom (12) made of quartz glass and having a nano coating.

## Description

### FIELD OF THE INVENTION

The invention relates to an arrangement and a method for studying samples and a container for samples.

### BRIEF DESCRIPTION OF THE INVENTION

An object of the invention is to provide a new apparatus and method for studying samples and container for samples.

The invention is characterized by the features of the independent claims. Embodiments of the invention are disclosed in the dependent claims.

According to an embodiment, an arrangement for studying samples comprises a frame providing a basis for at least two samples. The frame may be provided with at least two light sources. The arrangement also comprises at least one substrate for samples. The arrangement further comprises at least two sensors for detecting how much the samples emit or absorb light. The samples may be illuminated by the light sources to activate the samples to emit light. Thereafter the sensors may detect how much the samples emit light. Some samples may emit light even without first illuminating them with light sources. Naturally, in such case light sources are not needed but the sensors are used for detecting how much the samples emit light. The samples may also be illuminated by the light sources and simultaneously the sensors may detect how much the samples absorb the light emitted by the light sources. With the arrangement the samples are studied in a versatile manner. With the arrangement the same sample may be studied using different wavelengths from the different light sources. Alternatively different samples may be studied simultaneously using the same wavelength. Furthermore, the intensity of the light may vary and the effect of the intensity may be studied. Furthermore, a light source may emit light either continuously or in a pulsed manner. The results of the sensors may be collected and analysed in real time.

The substrate may comprise a bottom that is made of quartz glass, fused quartz or fused silica or any other optically transparent material. The substrate may be coated with a nano coating. The coating may be antibacterial, hydrophobic etc. Thus, the coating may effect on the sample. The arrangement may comprise at least two substrates having different coatings whereby the effect of the coating on the sample may be studied simultaneously.

The substrate may comprise a side wall that is detachable from the bottom. The side wall may be disposable whereby the bottom may be reused. The side walls may have different colours such that a first colour corresponds to a first coating of the bottom and a second colour corresponds to a second coating of the bottom etc. Thus the colour code of the side wall indicates the coating of the bottom of the substrate.

### BRIEF DESCRIPTION OF THE DRAWINGS

In the following, the solutions will be described in greater detail by means of preferred embodiments and with reference to the accompanying drawings, in which
Figure 1 is a schematic top view of an arrangement for studying samples,
Figure 2 is a schematic side view of the arrangement shown in Figure 1 and in cross-section along line A - A in Figure 1,
Figure 3 is a cross-sectional side view of a substrate,
Figure 4 is a side view of a temperature controller in cross section and
Figure 5 is a top view of a temperature controller shown in Figure 4.

### DETAILED DESCRIPTION OF THE EMBODIMENTS

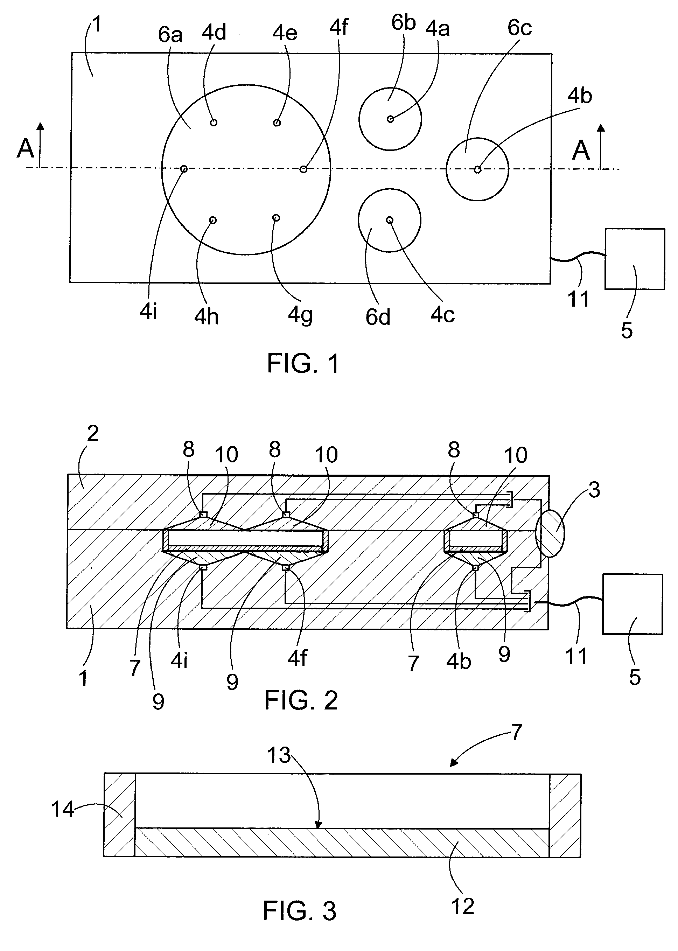

Figures 1 and 2 show an arrangement for studying samples. For the sake of clarity Figure 1 shows only a frame 1 of the arrangement but a cover 2 and a hinge 3 above the frame 1 are not illustrated. The cover 2 and the hinge 3 are, however, shown in Figure 2.

The frame 1 provides a basis for at least two samples. The frame 1 may be provided with light sources 4a - 4i. The light sources 4a - 4i may be LEDs, for example. Each light source 4a - 4i may emit light having a similar wavelength. Alternatively, the wavelength of the light of one light source may differ from the wavelength of the light of another light source. Furthermore, the intensity of the light sources may be similar or the intensity of one light source may differ from the intensity of another light source. It is also possible to form the light sources such that their wavelength and/or intensity is controlled by a control unit 5.

The frame 1 comprises cavities 6a - 6d. The light sources 4a - 4i are positioned in the bottom of the cavities 6a - 6d. Each cavity may comprise one light source as shown in connection with cavities 6b - 6d. Alternatively, one cavity may comprise two or more light sources as shown in connection with cavity 6a.

A substrate 7 is positioned in each cavity. The sample to be studied is positioned on the substrate 7. The substrate 7 may be a conventional Petri dish for example. Alternatively the substrate 7 may have a structure as disclosed in connection with Figure 3, for example. Each substrate 7 may hold one sample or alternatively one or more of the substrates 7 may hold two or more samples each.

Sensors 8 are provided in the cover 2. The sensor 8 may be a photodiode type sensor, for example. The sensors 8 may detect how much the sample under study absorbs the light emitted by the light source. There may be a corresponding sensor 8 for each light source 4a - 4i. The sensors 8 may be equipped with parts for improving the sensor sensitivity, such as filters or lenses.

The light sources 4a - 4i may also be used for illuminating the sample to activate the samples to emit light. In such case the sensors 8 may detect how much the sample emits light.

Some samples may be self-luminous. Thus, these samples may emit light even though they are not illuminated by an external light source, whereby, light sources are not necessarily needed at all. In such case, also, the sensors 8 may detect how much the samples emit light.

Each light source 4a - 4i may be provided with a dispersing lens 9 for dispersing the light emitted by the light source to a larger area to illuminate the sample move effectively. Correspondingly, each sensor 8 may be provided with a converging lens 10 for collecting the light emitted by the light source and passed through the sample.

As shown in Figure 2, the cover 2 may be connected to the frame by the hinge 3. Alternatively the hinge 3 may be omitted, whereby the cover and the frame may be separated from each other. In such a case a cable 11 to the control unit 5 is connected by a connector to the cover 2.

The cover 2 and the frame 1 may comprise mechanical forms for aligning the cover 2 and the frame 1. Aligning the cover 2 and the frame 1 means that each sensor 8 is on a desired location with respect to a corresponding light source 4a- 4i. The cover 2 and the frame 1 may, for example, comprise pins and holes such that when each pin is positioned to a corresponding hole the cover 2 and the frame 1 are aligned.

Figure 3 shows an example of a substrate 7. The substrate 7 may comprise a bottom 12. The bottom 12 may be a flat plate that is made of quartz glass, fused quartz or fused silica or any other optically transparent material. The bottom 12 comprises a coating 13 on its upper surface. The coating 13 is a nano coating. The nano coating may be an antibacterial nano coating or a hydrophobic nano coating, for example. Thus, the coating 13 may effect on the sample. The coating 13 may promote or impede the growth of the sample. Furthermore, the coating 13 may bring out or emphasize certain subjects or phenomena.

The coating 13 may be applied by an Atomic Layer Deposition (ALD) method or by a Molecular Beam Epitaxy (MBE) method, for example. Thus, the coating may be a suitable nano coating applied by the ALD method or the coating may be a suitable nano coating applied by the MBE method, for example.

An antibacterial nano coating may be formed by using silver, copper, or copper alloys such as brass, bronze, cupronickel, copper-nickel-zinc etc., for example. Furthermore, non-metallic antibacterial nano coatings may be formed by using organosilicon compounds, for example.

Hydrophobic nano coatings may be formed by using fluoropolymers, for example. Furthermore, a hydrophobic surface may be achieved by modifying the surface roughness by the coating method.

A quartz glass has excellent optical properties, it is inert and it tolerates high temperatures. Tolerating high temperatures is important if a nano coating is provided on the bottom at high temperature. A bottom 12 made of quartz glass may be reused by removing the coating using vaporization, for example.

The substrate 7 comprises, in addition to the bottom 12, a side wall 14. The side wall 14 may be detachable from the bottom 12. The side wall 14 may be disposable whereby the side wall 14 may be separated from the bottom 12 and the bottom 12 may be reused.

The substrate 7 is thus a container for holding the sample to be studied. Light may pass through the container. The container has the bottom and the sidewall and thereby the inner volume of the container is large enough for holding also non-solid samples. The container also has an inner volume large enough for enabling the sample to grow in the container.

The side wall 14 is preferably made of a neutral material. Neutral material means that it does not affect on the sample to be studied. An example of a neutral material is silicone. Also, other elastomers may be used as the neutral material.

The side wall 14 may have a certain color that corresponds to a certain coating 13. Thus according to an embodiment the substrate 7 may be made such that one side wall 14 has a first color that corresponds to a first coating 13 of the bottom 12 and another side wall 14 has a second color that corresponds to a second coating 13 of the bottom 12 etc. Thus the color or color code of the side wall 14 indicates the specific coating 13 of the bottom 12 of the substrate 7.

The sensor 8 detects light emitted by a light source and passed through the sample. Thus, the sensor 8 detects how much the sample absorbs the light. An absorption spectrum or an absorption amount correlates with the properties of the sample.

If at least two light sources emit the same wavelength it is possible to study two different samples simultaneously. According to an embodiment if at least two light sources emit light on different wavelengths it is possible to study how different wavelengths affect on the same sample.

It is also possible to emit light using different intensity and thereby study how the light intensity affects on the sample.

A light source may emit light on a continuous mode or on a pulsed mode, for example. On a continuous mode the light source emits light continuously when the light source is switched on. On a pulsed mode the light source emits light in a pulsed manner when the light source is switched on.

Also the coating of the substrate may be different it different substrates. Thus the effect of the substrate to the sample may be studied.

The results of the sensors may be collected and analyzed in real time. The result of the sensors may be transmitted to the control unit 5 using cable 9 as shown in Figures 1 and 2 or alternatively wireless communication may also be used. The results may be analyzed in the control unit 5 or the result may be further transferred to another unit for studying the results.

According to an embodiment the cavities 6b - 6d comprising only one light source are provided with light sources emitting light on different wavelengths. The cavity 6a comprising a plurality of the light sources may be provided with a mixture of the above mentioned light sources which means that two of the light sources emit light on a first wavelength, further two of the light sources emit light on a second wavelength and the rest two light sources emit light on a third wavelength.

In an embodiment a cavity comprises light sources emitting light on at least two different wavelengths. Such a solution provides a possibility to study if the sample has a tendency to seek its way towards one wavelength and/or to reject another wavelength, for example. Thus, the sample may freely choose a pleasing or satisfying wavelength

The studied sample may be a bacterium, for example. It is possible to add an antibiotic to the sample. The sample may be studied before and after adding the antibiotic. The studied sample may also be a plant or a cell culture, for example. It is possible to study the growth of the sample, for example.

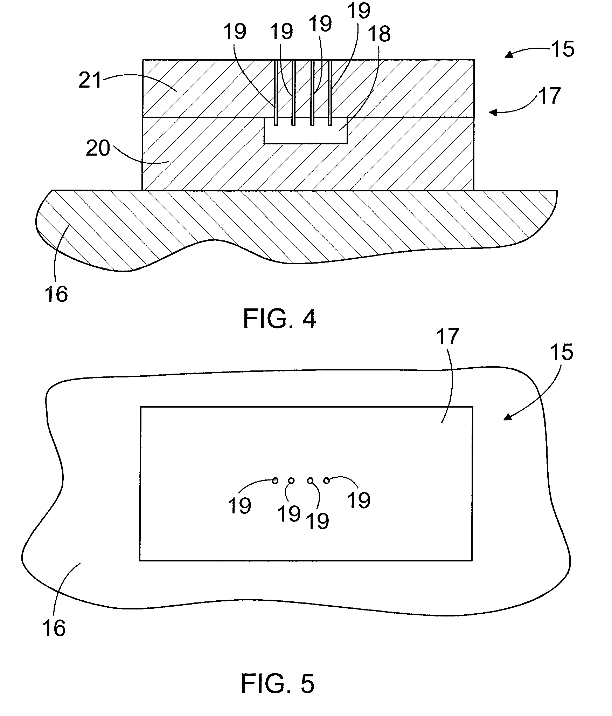

Figures 4 and 5 show a temperature controller 15. The temperature controller 15 observes the temperature and permanently indicates if the temperature has exceeded a certain limit or if the temperature has went below a certain limit. The temperature controller 15 may control the temperature of room or of a space and/or the temperature controller may control the temperature of the product or article 16 whereby the temperature controller 15 may be attached to the article 16.

As an example the temperature controller 15 is connected to the article 16. If during transport or storage, for example, the temperature has exceeded a certain limit the temperature controller 15 indicates that an exceeding has occurred although the temperature would have returned to an acceptable level later. Thus, a permanent change remains in the temperature controller. Thus, an example of a solution is to control that during transport or storage of frozen food the temperature has not exceeded an acceptable value.

The temperature controller 15 comprises a frame 17 made of plastics, for example. Inside the frame 17 there is a chamber 18. The size of the chamber may be a few milliliters, for example. The chamber 18 is filled with material that is solid under a certain temperature value and melts or liquefies above this certain temperature value. Capillary pipes or capillary tubes 19 extend out from the chamber 18. The inner diameter of the capillary tubes 19 may be one millimeter, for example.

If the temperature exceeds the certain value the material inside the chamber 18 liquefies and the capillary tubes 19 suck the liquid material inside the capillary tubes 19. The capillary tubes 19 keep the material inside them although the temperature returns below the above mentioned certain value.

Thus, if the capillary tubes 19 comprise material from the chamber 18 the temperature controller 15 indicates that the temperature has exceeded the acceptable limit value.

The material inside the chamber 18 is a phase change material. The material may be paraffin or paraffin compound, for example. Below a certain temperature the material is solid but above the certain temperature the phase of the material changes from solid to liquid.

The certain temperature value thus depends on the properties of the material inside the chamber 18. The properties of the material are adjusted by adjusting the proportions of the substances in the compound.

The frame 17 may comprise a lower part 20 and an upper part 21. The lower part 20 may comprise the chamber 18. The upper part 21 may comprise the capillary tubes 19. The capillary tubes 19 may extend in to the chamber such that the lower ends of the capillary tubes 19 are below the upper edge of the chamber 18 whereby it is ensured that the capillary tubes 19 suck the liquefied material.

The temperature controller may be manufactured such that the material is positioned inside the chamber in the lower part 20 and thereafter the upper part 21 is positioned on the lower part 20 and the upper part 21 is attached to the lower part 20.

In this description it is thus presented a temperature controller comprising a frame, a chamber inside the frame and a capillary tube extending out from the chamber, the chamber comprising phase change material.

In this description it is also presented a method for controlling temperature, the method comprising providing a temperature controller comprising a frame having a chamber and a capillary tube extending out from the chamber, the chamber comprising phase change material whereby exceeding a certain temperature value causes the phase change material to change its phase from solid to liquid the capillary tube sucking the liquid material inside the capillary tube, material inside the capillary tube thereby indicating that the temperature has exceeded the certain temperature value.

According to an embodiment the temperature controller comprises at least two capillary tubes extending out from the chamber.

According to another embodiment the capillary tube extends into the chamber such that the lower end of the capillary tube is below the upper edge of the chamber.

It will be obvious to a person skilled in the art that, as technology advantages the inventive concept can be implemented in various ways. The invention and its embodiment are not limited to examples describes above but may vary within the scope of the claims.

## Claims

1. An arrangement for studying samples comprising a frame (1) providing a basis for at least two samples,
at least one substrate (7) for samples,
at least two light sources (4a - 4i) for emitting light such that the light emitted by the light source (4a - 4i) passes through the sample and
at least two sensors(8) for detecting how much the samples emit or absorb light.

2. An arrangement as claimed in claim 1, wherein at least two light sources (4a - 4i) emit light on the same wavelength.

3. An arrangement as claimed in claim 1 or 2, wherein at least two light sources (4a - 4i) emit light on different wavelengths.

4. An arrangement as claimed in any one of the preceding claims, wherein an intensity of one light source (4a - 4i) differs from an intensity of another light source (4a - 4i).

5. An arrangement as claimed in any one of the preceding claims, wherein the substrate (7) comprises a bottom (12) made of quartz glass and having a nano coating.

6. A method for studying samples the method comprising providing a basis for at least two samples
providing at least one substrate (7) for samples,
providing at least two samples on the substrate (7),
illuminating the samples such that light passes through the samples
and
detecting how much the samples emit or absorb light.

7. A container for holding samples to be studied the container comprising a bottom and a sidewall whereby at least the bottom is made of optically transparent material and has a nano coating.

8. A container as claimed in claim 7, wherein the bottom is made of quartz glass.

9. A container as claimed in claim 7 or 8, wherein the nano coating is antibacterial and/or hydrophobic.

10. A container as claimed in any one of claims 7 to 9, wherein the sidewall is detachable from the bottom.

11. A container as claimed in claim 10, wherein the sidewall has a certain colour that is arranged to correspond to a certain coating of the bottom.
